# EUROPEAN PATENT SPECIFICATION

(11) **EP 2 285 214 B1**
(45) Date of publication and mention of the grant of the patent: **16.05.2012**
(21) Application number: 09762890.3
(22) Date of filing: 10.06.2009
(51) Int. Cl.: A61K 31/135, A61K 9/48

(54) **RASAGILINE SOFT GELATIN CAPSULES**
RASAGILIN-WEICHGELATINEKAPSELN
CAPSULES MOLLES DE RASAGILINE

(30) Priority: 10.06.2008 US 131566
(43) Date of publication of application: 23.02.2011
(73) Proprietor: Teva Pharmaceutical Industries Ltd., 49131 Petah Tiqva (IL)
(72) Inventor: SAFADI, Muhammad, Nazareth 16164 (IL); LICHT, Daniella, Givat Shmuel 54051 (IL); ZHOLKOVSKY, Marina, Bat-Yam 59496 (IL); CACIULARU, Fanny, Petach-Tikva 49491 (IL); ANDRYSEK, Tomas, Branka u Opavy 747 41 Hradec Nad Moravici (CZ); VRANA, Ales, 74707 Opava (CZ); ELBLOVA, Marie, CZ-747 82 Stablovice (CZ); STONIS, Roman, CZ-747 05 Opava (CZ)
(74) Representative: Nachshen, Neil Jacob
(86) International application number: PCT/US2009/003488
(87) International publication number: WO 2009/151594

(56) References cited:
- WO-A2-2007/044437
- US-A1- 2004 127 577
- US-A1- 2006 018 957
- US-A1- 2006 182 796
- US-A1- 2007 212 411
- US-A1- 2008 107 729

## Description

### Background of the Invention

United States Patents 5,532,415, 5,387,612, 5,453,446, 5,457,133, 5,599,991, 5,744,500, 5,891,923, 5,668,181, 5,576,353, 5,519,061, 5,786,390, 6,316,504, 6,630,514 disclose R(+)-N-propargyl-1-aminoindan ("R-PAI"), also known as rasagiline. Rasagiline has been reported to be a selective inhibitor of the B-form of the enzyme monoamine oxidase ("MAO-B") and is useful in treating Parkinson's disease and various other conditions by inhibition of MAO-B in the brain.

United States Patent No. 6,126,968 and PCT International Publication No. WO 95/11016, disclose pharmaceutical compositions comprising rasagiline.

PCT International Publication No. WO 2006/014973, discloses pharmaceutical compositions comprising rasagiline.

A concern in using monoamine oxidase ("MAO") inhibitors is the risk of hypertensive crises, often called the "cheese effect." (Simpson, G.M. and White K. "Tyramine studies and the safety of MAOI drugs." J Clin Psychiatry. 1984 Jul; 45 (7 pt 2): 59-91.) This effect is caused by inhibition of peripheral MAO. A high concentration of peripheral MAO is found in the stomach.

A further concern in Parkinson's disease patients is that many patients suffer from delayed gastric emptying (Pfeiffer, R. F. and Quigley, E. M. M. "Gastrointestinal motility problems in patients with Parkinson's disease: Epidemiology, pathophysiology, and guidelines for management," CNS-Drugs, 1999, 11(6): 435-448; Jost, W. H., "Gastrointestinal motility problems in patients with Parkinson's disease: Effects of antiparkinsonian treatment and guidelines for management", Drugs and Aging, 1997, 10(4): 249-258). Delayed gastric emptying (prolonged gastric residence) can be a cause of increased inhibition of peripheral MAO, and can contribute to the cheese effect.

### Summary of the Invention

The subject invention provides a pharmaceutical composition comprising a liquid fill which includes an amount of rasagiline mesylate, a shell comprising gelatin surrounding the liquid fill, and an enteric coating surrounding the shell.

### Brief Description of the Figures

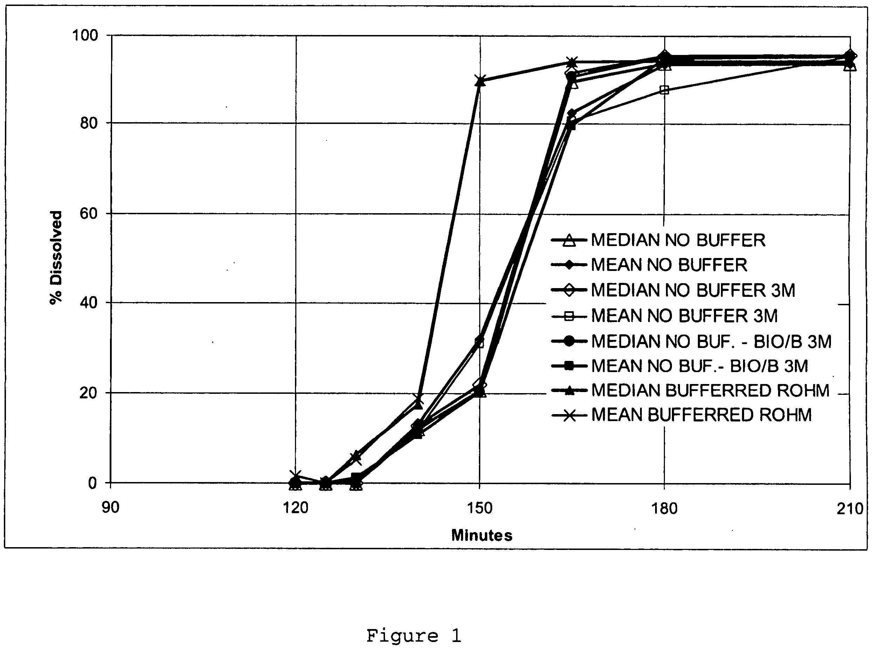

Figure 1 - Comparison of dissolution in pH=6.2, BUFFERRED vs. NO BUFFERRED. Figure 1 shows typical time-course of dissolution of enteric-coated capsules with improved dissolution characteristics - by the use of a proper plasticizer and subcoat - and without modification of the gelatin shell (no increase of ionic strength, no buffering).

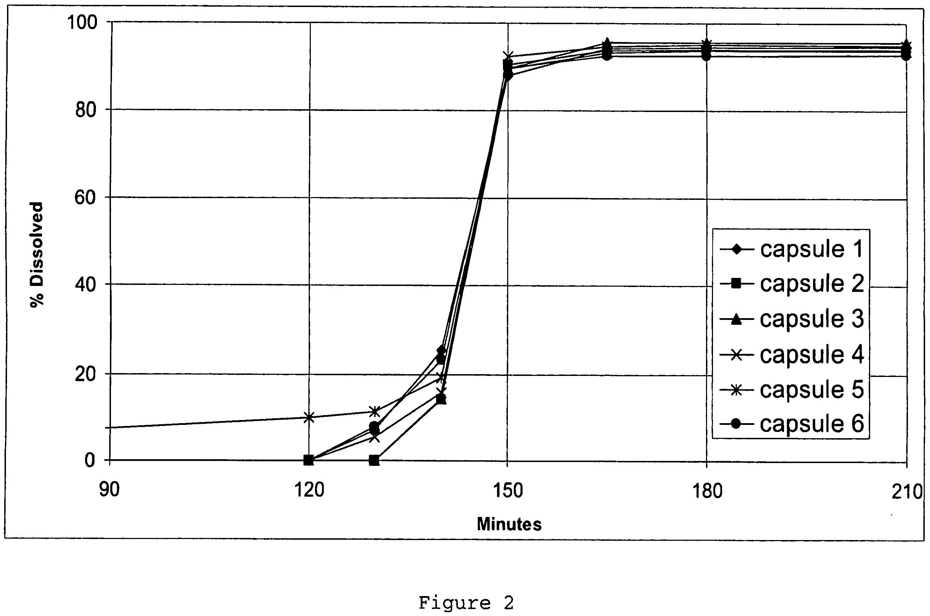

Figure 2 - Double coat, MODIFIED, 6.0mg/cm2, pH=6.2. Figure 2 shows typical time-course of dissolution of enteric-coated capsules with improved dissolution characteristics - by the use of a proper plasticizer and subcoat - and with modification of the gelatin shell (with increase of ionic strength, buffering to pH 8.5).

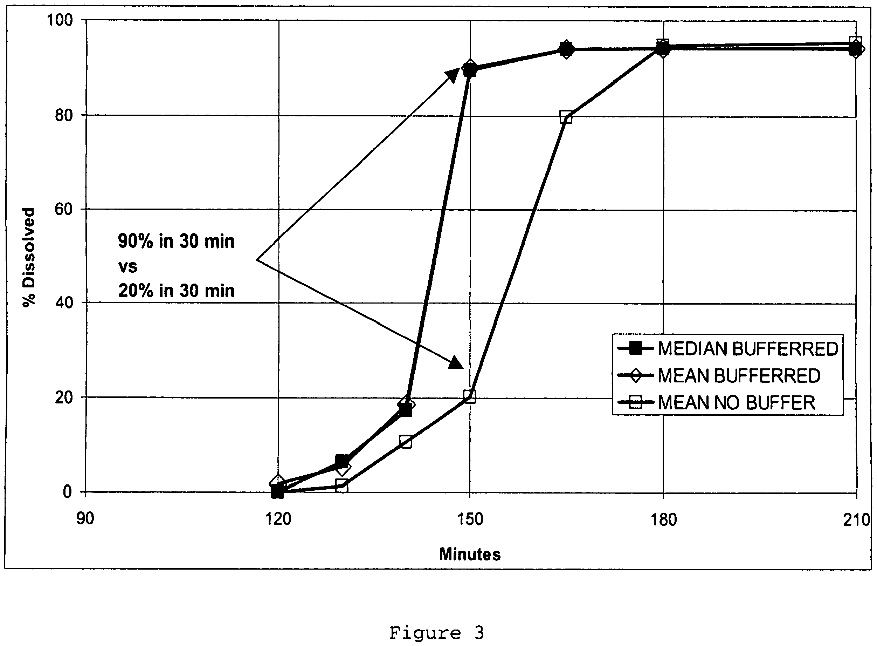

Figure 3 - Double coat, MODIFIED vs. Double coat, NO MODIFIED, pH=6.2. Figure 3 shows comparison of typical time-courses of dissolution of enteric-coated capsules without and with improved dissolution characteristics (without increase of ionic strength, NO buffering buffering versus with increase of ionic strength, buffering to pH 8.5).

### Detailed Description of the Invention

The subject invention provides a pharmaceutical composition comprising a liquid fill which includes an amount of rasagiline mesylate, a shell comprising gelatin surrounding the liquid fill, and an enteric coating surrounding the shell.

In an embodiment of the pharmaceutical composition, the liquid fill further comprises a hydrophilic or amphiphilic solvent or surfactant.

In another embodiment, the hydrophilic or amphiphilic solvent or surfactant is selected from the group consisting of: polyethylene glycol, propylene glycol, polyoxyethylene sorbitan fatty acid esters, polyoxyethylene castor oil derivatives and ethanol.

In yet another embodiment, the hydrophilic solvent is polyethylene glycol.

In yet another embodiment, the hydrophilic solvent is polyethylene glycol 400.

In another embodiment of the pharmaceutical composition, the pharmaceutical composition is free of propylene glycol.

In yet another embodiment of the pharmaceutical composition, the liquid fill further comprises an anti-oxidant.

In an embodiment, the antioxidant is water-soluble.

In another embodiment, the antioxidant is selected from the group consisting of: propyl gallate, BHA, BHT and ascorbic acid.

In yet another embodiment, the antioxidant is BHA.

In yet another embodiment of the pharmaceutical composition, the shell further comprises a plasticizer.

In an embodiment, the plasticizer is selected from the group consisting of glycerol and sorbitol or a combination thereof.

In yet another embodiment of the pharmaceutical composition, the enteric coating comprises Poly(methacrylic acid, ethyl acrylate) 1 : 1.

In an embodiment, the enteric coating further comprises a plasticizer.

In another embodiment, the plasticizer is polyethylene glycol 20,000.

In yet another embodiment of the pharmaceutical composition, a non-enteric subcoat is present between the gelatin shell layer and the enteric coating layer.

In an embodiment, the total weight of the subcoat and enteric coating layer is less than 10% of the total capsule weight.

In another embodiment, the weight of the enteric coating layer is less than 8% of the total capsule weight.

In yet another embodiment, the weight of the enteric coating layer is less than 6% of the total capsule weight.

In yet another embodiment, the weight of the enteric coating layer is less than 4% of the total capsule weight.

In yet another embodiment, the gelatin shell layer comprises a cross-linking inhibitor.

In yet another embodiment, cross-linking inhibitor in the gelatin shell layer is glycine.

In yet another embodiment, the subcoat comprises hydroxypropyl methyl cellulose.

In another embodiment of the pharmaceutical composition, when placed in a basket apparatus in 500 mL of aqueous buffered solution at a pH of 8.2 at 75 revolutions per minute, not less than 85% of the rasagiline in the pharmaceutical composition is released into solution within 30 minutes.

The subject invention also provides a pharmaceutical composition comprising a liquid fill which includes an amount of rasagiline mesylate, a shell comprising gelatin surrounding the liquid fill, and an enteric coating surrounding the shell, when placed in a basket apparatus in 500 mL of aqueous 0.1 N HCl at 37°C at 75 revolutions per minute, not more than 10% of the rasagiline is released into solution in 120 minutes and when the composition is subsequently placed in a basket apparatus in 500 mL of aqueous buffered solution at a pH of 6.8 at 37°C at 75 revolutions per minute, not less than 75% of the rasagiline is released into solution within 90 minutes.

In an embodiment of the pharmaceutical composition, when placed in a basket apparatus in 500 mL of aqueous 0.1 N HCl at 37°C at 75 revolutions per minute, not more than 10% of the rasagiline is released into solution in 120 minutes and when the composition is subsequently placed in a basket apparatus in 500 mL of aqueous buffered solution at a pH of 5.2 at 37°C at 75 revolutions per minute, not more than 10% of the rasagiline is released into solution within 90 minutes.

The subject invention further provides a pharmaceutical composition comprising a liquid fill which includes an amount of rasagiline mesylate, a shell comprising gelatin surrounding the liquid fill, and an enteric coating surrounding the shell, when placed in a basket apparatus in 500 mL of aqueous 0.1 N HCl at 37°C at 75 revolutions per minute, not more than 10% of the rasagiline is released into solution in 120 minutes and when the composition is subsequently placed in a basket apparatus in 500 mL of aqueous buffered solution at a pH of 6.2 at 37°C at 75 revolutions per minute, not less than 75% of the rasagiline is released into solution within 45 minutes.

MAO inhibitors that selectively inhibit MAO-B are largely devoid of the potential to cause the "cheese effect". Nonetheless, the possibility exists that delayed gastric emptying of R-PAI may contribute to this phenomenon. Therefore, a main goal in developing the formulations of the current invention was to develop a delayed release, enteric coated formulation comprising rasagiline mesylate in an amount equivalent to 1 mg of rasagiline base which would release the active ingredient in the duodenum and the jejunum, past the stomach.

### Development of Delayed Release Formulation

During the development of the formulations of the current invention, it was determined that the formulations should meet the criteria of bioequivalence to the known, immediate release rasagiline mesylate formulations (as disclosed in example 1) in a single dose bio-equivalence study in healthy subjects. These criteria include similarity of Cₘₐₓ and AUC₀₋ₜ (area under the curve) within the range of 80-125% within a 90% confidence interval between the new formulations and the known, immediate release formulations. The difference between the two formulations should be evident in bioequivalence studies as a difference in tₘₐₓ. In other words, the mean pharmacokinetic profile of the formulations of the current invention should match the mean pharmacokinetic profile of the formulations of the known immediate release formulation, with the exception of the tₘₐₓ which should be greater for the delayed release formulation than for the immediate release formulation.

The reason for attempting to match the mean Cₘₐₓ and AUC₀₋ₜ of the known immediate release formulation (i.e. to formulate a delayed release formulation that is bioequivalent) is that the efficacy of the immediate release formulation has been proven, and it is likely that the efficacy of the formulation relates to its mean Cₘₐₓ and/ or AUC. (Arch Neurol. 2002; 59:1937-1943.)

In order to reach this target, development was directed toward enteric coated capsules having a liquid fill, filled with rasagiline mesylate, with an enteric coating which allows release of the rasagiline mesylate in a specific range of pH. This specific pH range would prevent the formulation to release rasagiline mesylate in the stomach, and would allow the formulation to release rasagiline mesylate quickly under the physiological conditions of the intestine.

In PCT application publication WO 2006/014973, enteric-coated rasagiline mesylate pharmaceutical formulations were disclosed. In the disclosed formulations (Example 1, 2 and 4) methacrylic acid - ethyl acrylate copolymer (1:1) 30% dispersion, known as Eudragit® L-30 D-55 was used. As evident in the above-mentioned publication, these formulations were indeed delayed-release formulations as shown by their dissolution profiles and by the in-vivo data, however, the pharmacokinetic profile, in terms of mean Cₘₐₓ did not match the pharmacokinetic profile of the immediate release rasagiline mesylate formulations.

The excipient methacrylic acid - ethyl acrylate copolymer (1:1) 30% dispersion, known as Eudragit® L-30 D-55, used in the above-mentioned publication WO 2006/014973, when applied as an aqueous dispersion either on tablets or on spheres prevents dissolution of the coated composition at low acidic pH. The structure of this polymer is as follows: 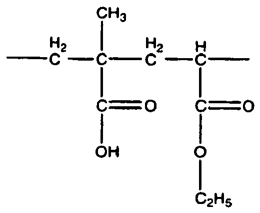 The ratio of the free carboxyl groups to the ester groups is approximately 1:1. The average molecular weight is approximately 250,000.

When this excipient is used in an aqueous dispersion or in an organic solution and formed into a film coating of a pharmaceutical formulation, it is intended to dissolve at a pH of about 5.5. (Aqueous Polymeric Coatings for Pharmaceutical Dosage Forms; Second Edition, Revised and Expanded. Ed. James W. McGinity, 1997.)

It is probable that these prior art formulations began to dissolve in the stomach, perhaps in the presence of food which can raise the pH in the stomach, and continued to dissolve over a prolonged period of time in the duodenum and the jejunum. The prolonged dissolution period could explain why the Cₘₐₓ of these prior art formulations was significantly lower than the Cₘₐₓ of the immediate release formulations to which they were compared. In addition, once the coated tablet formulations began to disintegrate, the rasagiline mesylate was not dissolved, therefore it was not available for immediate absorption in the intestine. An advantage of the formulation of the instant invention is that the rasagiline mesylate is already dissolved in solution within the capsule, so once the capsule passes into the intestine and the pH rises, the capsule will rapidly break open, releasing dissolved rasagiline mesylate into the intestine, thereby allowing for rapid absorption. Achieving the goal of a delayed-release pharmaceutical formulation in which the Cₘₐₓ is similar to the corresponding immediate-release formulation is not trivial. In general, when delayed release formulations are compared to their immediate release counterparts in bio-studies, the Cₘₐₓ of the delayed release formulations are lower than the Cₘₐₓ in the corresponding immediate release formulations. (Mascher, et al. Arneimittelforschung. 2001; 51(6): 465-9. Behr, et al. J. Clin Pharmacol. 2002; 42(7): 791-7.)

### Soft Gelatin Capsules

Gelatin capsules, used as a pharmaceutical dosage form or with food supplements, consist of a gelatin shell surrounding a core filled with the composition being delivered. Hence, gel capsules may be a hard capsule, filled with solid or semi-solid fill and formed by two partial moieties of the shell, which are joined in order to create the final shell of the capsule, or a soft capsule, where a liquid or semi-liquid fill is encapsulated by a one piece gelatin shell, and optionally, even so-called caplets, where a modified tablet is covered by gelatin shell.

Soft gelatin capsules are produced by injecting the liquid or semi-liquid fill between two gelatin strips, either by discontinuous formation or by continuous formation (i.e., rotary-die process). During the manufacturing process, both strips of the gelatin gel have to be lubricated by a suitable lubricant in order to avoid early sticking of the fresh gelatin gel to the machine parts or to each other. Suitable lubricant agents include pharmaceutical oils, as for example mineral oils (paraffin oil), synthetic oils (silicone oil) or vegetable oils (coconut oil, corn oil).

Liquid or semi-liquid fills for the soft gelatin capsules are divided into two basic groups according to their miscibility with water (Hom and Jimerson, Capsules, Soft. In: Encyclopedia of Pharmaceutical Technology. Vol. 2, Swabrick and Boylan (Eds), Marcel Dekker, New York and Basel, 269-284, 1990; and Lachmann, Theory and Practice of Industrial Pharmacy, 2nd Ed. Lea and Febiger, Philadelphia, 1986):
1. Capsules with water immiscible liquids, as for example, vegetable and aromatic oils, aromatic and aliphatic hydrocarbons, chlorohydrocarbons, ethers, esters, alcohols and high molecular organic acids; and
2. Capsules with water miscible liquids, as for example, polyethyleneglycols and non-ionogenic surfacial active material (surfactants).

The fills containing only the compounds from the first group do not mix with water. After their encapsulation into the fresh gelatin gel, the excess water leaves the gel and enters the inner fill. The excess water is almost completely resorbed by the gelatin shell of the capsule during the drying process, until equilibrium with the surroundings is achieved. Conversely, fills containing compounds from the second group are able to take in and absorb a certain amount of water, which can enter the fill of the capsule after its encapsulation. The resorption process is more difficult for these fills and equilibrium achievement is conditioned by the HLB (hydrophilic to lipophilic balance) value and absorption hysteresis curve for gelatin shells of concrete composition (York, J. Pharm Pharmacol. 33:269-273, 1981).

Besides the two groups of fills for soft gelatin capsules mentioned above, there exists another group of compounds or their mixtures, which are totally inappropriate for being filled into gelatin capsules.

Both the capsules with water immiscible liquids and the capsules with water miscible liquids can be enteric-coated for drug delivery to the small intestine. As the lipophilic drugs formulations in water immiscible liquids are rarely susceptible to decomposition in the acidic environment of the stomach, the enteric coating is especially suitable for drugs that have their absorption window rather small (in the proximal intestine), or, which are susceptible to decomposition in acidic environment of the stomach, and concurrently are formulated in water miscible liquids, as for example, polyethyleneglycols and non-ionogenic surfacial active material (surfactants).

Normally, enteric-coated products for drug delivery to the small intestine dissolve rapidly in the *in-vitro* dissolution tests (in 40-60 min). However, there are two major limitations of practical use of enteric-coated products for drug delivery to the small intestine:
1. the situation in vivo frequently does not reflect the in vitro behaviour, so that such enteric-coated products may take up to 2 hours to disintegrate in *in-vivo* conditions; and
2. for those drugs which have their absorption window in the proximal (upper) small intestine, a rapid disintegration of the solid dosage form is required after the pass through stomach.

Numerous factors affect the dissolution of enteric-coated solid dosage forms, namely tablets and hard gelatin capsules, can be affected by the thickness of the coating polymer, by the appropriate selection of the plasticizer for the coat, and by using two coating layers (the subcoat and the upper coat).

On the other hand, the use of soft or hard gelatin capsules is often affected by the risk of gelatin cross-linking. The employment of gelatin capsules as an oral delivery means is known in the pharmaceutical arts. In pharmaceutical applications, soft gelatin capsules are especially suitable for oral administration of lipophilic active substances. However, once the cross-linking of the gelatin occurs, the gelatin shell becomes less soluble in an aqueous medium, especially in an acidified water medium. The cross-linking delays the disintegration of the gelatin shell, which subsequently delays the dissolution of the inner content of the capsule as compared with a similar capsule not exposed to long time storage or stress-conditions which promote cross-linking.

Therefore, it is necessary when the gelatin capsule contains a component which promotes cross-linking in the gelatin shell to prepare a formulation which will not induce delayed disintegration and/or delayed dissolution of the inner content of the capsule following storage or after exposure to stress conditions.

The shells of both hard and soft gelatin capsules are susceptible to cross-linking. Cross-linking has been demonstrated by a prolongation of the dissolution time and release of drug substance. The delay is attributed to only partial dissolution of the gelatin shell (in case of soft gelatin capsules, the dissolved part is the outer layer of shell). In some instances, the inner layer of the gelatin shell forms a thin film, called a pellicle, which remains intact and envelopes the inner volume of the capsules. This effect is described by Carstensen and Rhodes (Drug Dev. Ind. Pharm, 19(20): 2709-2712, 1993) or Bottom, et al., (J. of Pharm. Sci., 86(9): 1057-1061, 1997).

Considering relatively small intensity of mixing in dissolution apparatus, the rupture of gelatin shell containing the pellicle is worsened and delayed as well and it is the cause of high variability of results of the dissolution test.

There are presently two basic methods described in the literature addressing the dissolution problems of soft gelatin capsules. These methods include:
(a) demonstrating that the altered dissolution profiles obtained from cross-linked gelatin capsules is a laboratory phenomenon by utilizing *in vivo* bioequivalence and/or clinical studies which attempt to demonstrate that actual biological availability of test agents are not negatively affected by the cross-linking; and
(b) elimination of the causes of cross-linking, namely (i) elimination of physical conditions, which promote the cross-linking, (ii) elimination of substances, which promote the cross-linking (cross-linking promoters), and (iii) addition of cross-linking inhibitors (where the most effective action is concurrent combination of these precautions).

The instant invention provides a solution to the problem of peripheral MAO inhibition by providing pharmaceutical dosage forms comprising rasagiline which are adapted to inhibit the release or absorption of rasagiline in the stomach (i.e. delay the release of rasagiline until at least a portion of the dosage form has traversed the stomach). This avoids or minimizes absorption of rasagiline in the stomach, thereby avoiding or minimizing the potential cheese effect.

In addition, the present invention provides a very effective way to prevent the cross-linking of gelatin in soft gelatin capsules by the use of cross-linking inhibitor and inhibitor enhancer.

In all of its aspects, the present invention provides an oral pharmaceutical dosage form useful for treating a condition selected from the group consisting of: Parkinson's disease, brain ischemia, head trauma injury, spinal trauma injury, neurotrauma, neurodegenerative disease, neurotoxic injury, nerve damage, dementia, Alzheimer's type dementia, senile dementia, depression, memory disorders, hyperactive syndrome, attention deficit disorder, multiple sclerosis, schizophrenia, and affective illness, but with a reduced risk of peripheral MAO inhibition that is typically associated with administration of rasagiline with known oral dosage forms.

Specific examples of pharmaceutically acceptable carriers and excipients that may be used to formulate oral dosage forms of the present invention are described, e.g., in U.S. Pat. No. 6,126,968 to Peskin et al., issued Oct. 3, 2000. Techniques and compositions for making dosage forms useful in the present invention are described, for example, in the following references: 7 Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol 40 (Gilbert S. Banker, Christopher T. Rhodes, Eds.).

Tablets may contain suitable binders, lubricants, disintegrating agents, coloring agents, flavoring agents, flow-inducing agents, melting agents, and plasticizers. For instance, for oral administration in the dosage unit form of a tablet or capsule, the active drug component can be combined with an oral, non-toxic, pharmaceutically acceptable, inert carrier such as lactose, gelatin, agar, starch, sucrose, glucose, methyl cellulose, dicalcium phosphate, calcium sulfate, mannitol, sorbitol, microcrystalline cellulose and the like. Suitable binders include starch, gelatin, natural sugars such as glucose or beta-lactose, corn starch, natural and synthetic gums such as acacia, tragacanth, or sodium alginate, povidone, carboxymethylcellulose, polyethylene glycol, waxes, and the like. Lubricants used in these dosage forms include sodium oleate, sodium stearate, sodium benzoate, sodium acetate, sodium chloride, stearic acid, sodium stearyl fumarate, talc and the like. Disintegrators include, without limitation, starch, methyl cellulose, agar, bentonite, xanthan gum, croscarmellose sodium, sodium starch glycolate and the like, suitable plasticizers include triacetin, triethyl citrate, dibutyl sebacate, polyethylene glycol and the like.

The basket-type apparatus used in this invention is the apparatus 1 described in the United States Pharmacopeia, 29th Edition, chapter 711. The apparatus is constructed as follows:

The assembly consists of the following: a covered vessel made of glass or other inert, transparent material; a motor; a metallic drive shaft; and a cylindrical basket.

The vessel is partially immersed in a suitable water bath of any convenient size or placed in a heating jacket. The water bath or heating jacket permits holding the temperature inside the vessel at 37 ± 0.5 during the test and keeping the bath fluid in constant, smooth motion. No part of the assembly, including the environment in which the assembly is placed, contributes significant motion, agitation, or vibration beyond that due to the smoothly rotating stirring element. Apparatus that permits observation of the specimen and stirring element during the test is preferable. The vessel is cylindrical, with a hemispherical bottom and with one of the following dimensions and capacities: for a nominal capacity of 1 L,

the height is 160 mm to 210 mm and its inside diameter is 98 mm to 106 mm; for a nominal capacity of 2 L, the height is 280 mm to 300 mm and its inside diameter is 98 mm to 106 mm; and for a nominal capacity of 4 L, the height is 280 mm to 300 mm and its inside diameter is 145 mm to 155 mm. Its sides are flanged at the top. A fitted cover may be used to retard evaporation. The shaft is positioned so that its axis is not more than 2 mm at any point from the vertical axis of the vessel and rotates smoothly and without significant wobble. A speed-regulating device is used that allows the shaft rotation speed to be selected and maintained at the rate specified in the individual monograph, within ±4%. Shaft and basket components of the stirring element are fabricated of stainless steel type 316 or equivalent.

Unless otherwise specified in the individual monograph, use 40-mesh cloth. A basket having a gold coating 0.0001 inch (2.5 µm) thick may be used. The dosage unit is placed in a dry basket at the beginning of each test. The distance between the inside bottom of the vessel and the basket is maintained at 25 ± 2 mm during the test.

Within the context of this invention, dissolution is measured as an average measurement of 6 pharmaceutical dosage forms, for example, capsules or tablets.

This invention will be better understood from the experimental details which follow. However, one skilled in the art will readily appreciate that the specific methods and results discussed are merely illustrative of the invention as described more fully in the claims which follow thereafter.

### Example 1: Rasagiline Immediate Release Tablets

Rasagiline immediate release tablets were prepared using the ingredients listed in Table 1.

**Table 1. Ingredients in Rasagiline Immediate Release Tablets**

| Ingredients | mg/tablet |
|---|---|
| Rasagiline mesylate | 1.56 |
| Mannitol USP | 78.84 |
| Colloidal Silicon Dioxide | 0.6 |
| Starch NF | 10.0 |
| Pregelatinized Starch NF/EP | 10.0 |
| Stearic Acid NF/EP | 2.0 |
| Talc USP/EP | 2.0 |

Rasagiline mesylate, mannitol, half of the colloidal silicon dioxide, starch and pregelatinized starch were mixed in a Diosna P-800 mixer for about 5 minutes. Water was added and the mixture was mixed further. The granulate was dried and the remainder of the colloidal silicon dioxide was added. The granulate was ground in a Frewitt mill and stearic acid and talc were added. The granulate was mixed for five minutes in a tumbler and was tableted.

### Example 2: Rasagiline capsules containing enteric coated particles

Rasagiline capsules were prepared according to example 3 in PCT application publication WO 2006/014973.

These capsules were tested for dissolution in 500 ml of various aqueous acidic media made from phthalate buffer adjusted to the target pH from 2.4 to 3.6 using HCl solution and adjusted to the target pH of 4.2 to 5.2 using NaOH solution.

**Table 2. Dissolution of capsules, in different pH media, in percent**

| Time (min) | pH 2.4 | pH 3.0 | pH 3.6 | pH 4.2 | pH 5.2 |
|---|---|---|---|---|---|
| 30 | 0 | 0 | 0 | 0 | 0 |
| 60 | 0 | 0 | 0 | 0 | 22 |
| 90 | 0 | 0 | 0 | 0 | 48 |
| 120 | 0 | 0 | 0 | 0 | 66 |

The capsule formulation begins to dissolve after 60 minutes in medium with a pH of 5.2. This may explain the lower Cmax value in a single dose, crossover comparative pharmacokinetic study in 12 healthy male volunteers in the fasting state attributed to this formulation when compared to the immediate release formulation of example 1. It is likely that the dissolution of this formulation occurs slowly from the time the formulation enters the duodenum until the formulation proceeds in the intestine to the jejunum. Without being bound by theory, this may be attributed to the fact that the capsule disintegrates in the stomach and the coated pellets travel at different speeds through the intestine, releasing the rasagiline over a longer period of time, over a larger intestinal surface area.

### Example 3: Fill of Rasagiline Mesylate Soft Gelatin Capsules

Solubility of Rasagiline mesylate was evaluated in various solvents in order to determine which solvents would be suitable for developing a fill for rasagiline mesylate soft gelatin capsules.

**Table 3: Solubility of Rasagiline mesylate in different solvents**

| Solvent | Conditions | Observation |
|---|---|---|
| Soybean oil | RT, stirring | Not soluble |
| | 37ºC, stirring | Not soluble |
| Miglyol 810 | RT, stirring | Not soluble |
| (Caprylic/Capric Triglyceride) | 37ºC, stirring | Not soluble |
| Labrafil M 1944 CS | RT, stirring | Not soluble |
| (polyoxyethylated glycolysed glycerides) | 37ºC, stirring | Not soluble |
| Gelucire 44/14 (Lauroyl macrogol-32 glyceride) | 45-50ºC, stirring | Not soluble |
| Labrasol (Caprylocaproyl macrogol-8 glyceride) | 37-40ºC, stirring | 10 mg/g soluble, clear solution |
| Capmul MCM (glyceryl mono- & dicaprate) | RT, stirring | 64 mg/g soluble, clear solution |
| Transcutol (2-(2-ethoxyethoxy)ethanol) | RT, stirring | 25 mg/g soluble, clear solution |
| Ethanol | RT, stirring | 66.6 mg/g soluble, clear solution |
| Tween 80 (polyoxyethylene sorbitan fatty acid esters) | RT, stirring | 10 mg/g soluble, clear solution |
| Cremophor EL (polyoxyethylene castor oil derivative) | RT, stirring | 25 mg/g soluble, clear solution |
| Polyethylene Glycol 400 | RT, stirring | 25 mg/g soluble, clear solution |
| Propylene Glycol | RT, stirring | 25 mg/g soluble, clear solution |

Rasagiline Mesylate was found to be soluble in solvents such as Caprylocaproyl macrogol-8 glyceride, glyceryl mono- & dicaprate, polyoxyethylene sorbitan fatty acid esters, 2-(2-ethoxyethoxy)ethanol, polyoxyethylene castor oil derivatives, polyethylene glycol, propylene glycol, and ethanol.

**Table 4: Stability of Rasagiline Mesylate in solution with various solvents**

| Rasagiline Mesylate was tested in solutions at accelerated conditions. The numbers represent percent rasagiline in solution by assay. | | | | | | | | |
|---|---|---|---|---|---|---|---|---|
| **RM in mg/g solvent** | **Solution** | **T=0** | **55ºC, 2 weeks** | **40ºC 75%RH 1 m** | **40ºC 75%RH 2 m** | **40ºC 75%RH 3 m** | **40ºC 75%(RH 4 m** | **40ºC 75%RH 6 m** |
| 5 mg/g | 100% Capmul MCM | 85.4 | 84.4 | 84.9 | 90.8 | 87.0 | 91.3 | 91.0 |
| 5 mg/g | 50% Capmul MCM, 50% Miglyol 810 | 84.4 | 92.6 | 91.3 | 100.9 | 99.1 | 100.6 | 101.6 |
| 5 mg/g | 17% Capmul MCM, 83% Miglyol 810 | 95.7 | 94.4 | 94.4 | 102.8 | 101.2 | 103.7 | 104.9 |
| 25 mg/g | PEG 400 | 98.9 | 93.1 | 95.8 | 93.3 | 93.5 | 84.0 | 82.8 |
| 25 mg/g | PG | 100.3 | 93.7 | 96.6 | 89.0 | 85.5 | 79.5 | 77.9 |
| 10 mg/g | Tween 80 | 100.5 | 95.6 | 97.3 | 94.6 | 93.5 | 93.8 | 91.4 |

Solutions with PEG, PG and Tween 80, hydrophilic solvent solutions, were shown to have a decrease in assay in accelerated conditions solvent solutions.

In parallel, four Rasagiline mesylate (1.56 mg per/capsule) solutions were prepared and Dissolution tests of hard gelatin capsules filled by these solutions were performed in two media: 0.1N HCl and buffer phosphate pH 6.8. The dissolution results are presented in the following tables:

**Table 5. Dissolution results**

| Conditions: Paddle, helix, 0.1N HCl, 500 ml, 50 rpm | | | | |
|---|---|---|---|---|
| Solvent | 5 min | 10 min | 15 min | 30 min |
| Capmul MCM/Miglyol | 96.7 | 99.8 | 99.8 | 99.8 |
| PEG 400/5%Glycerin | 98 | 99 | 99 | 99 |
| PEG 400/10%PG | 93 | 93 | 93 | 93 |
| PEG 400/10%Tween 80 | 96 | 98 | 98 | 98 |

**Table 6. Dissolution Results**

| Conditions: Paddle, helix, buffer phosphate pH 6.8, 500 ml, 50 rpm | | | | |
|---|---|---|---|---|
| Solvent | 5 min | 10 min | 15 min | 30 min |
| Capmul MCM/Miglyol | 56.1 | 79.6 | 85.8 | 90.5 |
| PEG 4005%/Glycerin | 71 | 88 | 93 | 94 |
| PEG 400/10%PG | 93 | 93 | 93 | 91.5* |
| PEG 400/10%Tween 80 | 80 | 91 | 92 | 92 |

No difference of dissolution results was observed in 0.1N HCl between the formulations, as dissolution was rapid. In buffer phosphate pH 6.8 solution the highest dissolution profile was observed for formulation included 10% PG and PEG 400. For hydrophobic solvent- based formulation much slower dissolution results were obtained.

Based on the enhanced dissolution of Rasagiline Mesylate hydrophilic solvents in phosphate buffer at pH of 6.8, it was decided to attempt to enhance stability of those formulations.

Based on the instability in hydrophilic solvents, it was decided that an antioxidant should be added to stabilize the formulations.

### Example 4: Fill of Rasagiline Mesylate Soft Gelatin Capsules with Propylene Glycol and Antioxidant

A solution was prepared using Rasagiline Mesylate in a concentration of 15.6 mg/g solvent, 10% ethanol, 0.01% BHT, and the remainder was propylene glycol. This solution was placed in accelerated conditions of 40°C for 1 month, and 55°C for 2 weeks. The solution was found to be stable, and the rasagiline mesylate concentration did not decrease.

A similar formulation was prepared, but without the BHT. This formulation was stable at 55°C for 2 weeks, but at 40°C for 1 month, the rasagiline mesylate concentration decreased by 3.6%.

This experiment indicates that anti-oxidants, such as BHT, can be useful in stabilizing solutions of rasagiline mesylate in hydrophilic solvents such as propylene glycol.

### Example 5: Fill of Rasagiline Mesylate Soft Gelatin Capsules with PEG 400 and Antioxidants

Additional solutions were prepared with additional antioxidants as in table 7. Their stability (rasagiline content by assay) is shown in table 8. Initial concentration of Rasagiline Mesylate in the solutions was 15.6 mg/g.

**Table 7**

| Solution | Solvent | Antioxidant |
|---|---|---|
| C | 0% PG, remainder PEG 400 | Propyl Gallate 0.1% |
| D | 10% PG, remainder PEG 400 | BHA 0.01% |
| E | 20% PG, remainder PEG 400 | Ascorbic acid 0.1% |

**Table 8**

| Solution | T=0 | 55ºC, 2 weeks | 40ºC, | 40ºC | 40ºC |
|---|---|---|---|---|---|
| | | | 1 m | 2 m | 3 m |
| C | 100.2 | 98.4 | 98.2 | 98.5 | 97.4 |
| D | 99.5 | 97.9 | 98.7 | 98.8 | 95.9 |
| E | 99.6 | 99.1 | 97.5 | 96.8 | - |

Rasagiline content by assay was found to be stable in solutions C and D. Rasagiline content of solution C decreased by 2.7% after 2 months storage at 40ºC, but the amount of impurities was significantly lower than it was without antioxidants.

Based on solution D, a new formulation was prepared with addition of Glycerin. Glycerin is used as an effective plasticizer for soft gelatin capsules containing hydrophilic fills due to the strong intermolecular interactions between the hydroxy- groups of glycerin and the hydrophilic groups on gelatin. Glycerin and water may migrate from the shell to the hygroscopic fill. In order to balance this migration process and prevent embrittlement of the shell glycerin was added to the filling solution.

Additional solutions were prepared as described in table 9. Their stability (rasagiline content by assay) is shown in table 10.

**Table 9**

| Solution | Solvent | Antioxidant |
|---|---|---|
| F | 10% PG, Glycerin 5%, remainder PEG 400 | BHA 0.01% |

**Table 10**

| Solution | T=0 | 40ºC, | 25ºC, | 40°C, | 25ºC, | 40ºC, | 25ºC, |
|---|---|---|---|---|---|---|---|
| | | 1M | 1M | 2M | 2M | 3M | 3M |
| F, batch 1 | 103.8 | 100.0 | 99.9 | 100.8 | 102.0 | 101.5 | 101.0 |
| F, batch 2 | 103.8 | 102.0 | 101.8 | 102.2 | 102.0 | 102.3 | 102.5 |

As shown in Table 10, solution F was found to be stable.

### Example 6: Rasagiline Soft Gelatin Capsules with Propylene Glycol

Gelatin Capsules were prepared using the following fill:

| Component | % |
|---|---|
| Rasagiline Mesylate | 1.04 |
| Glycerol, 85% | 5.88 |
| Propylene Glycol | 10.00 |
| PEG 400 | 83.07 |
| BHA | 0.01 |

The soft gelatin capsule shell was made from the following excipients:

| Component | % |
|---|---|
| Gealtina 150 bloom, var. B, | 45.20 |
| Glycerol 85% PhEur | 18.0 |
| Sorbitol 70%, noncrystalline PhEur | 4.00 |
| Glycine, USP | 0.5 |
| Purified Water | 30.3 |
| Propylene Glycol | 2.0 |

Capsule preparation itself consists of three steps - fill preparation, gelatin preparation and encapsulation of the fill preparation into the gelatin forming soft gelatin capsules.

### Fill preparation:

Under ambient condition in appropriate container Propylene Glycol 400, Glycerol 85% and Buthylhydroxyanizol, were mixed and Rasagiline Mesylate was added and mixed with the mixer for approximately 30 min to obtain a clear yellowish solution. Then the solution was filtrated through a 5 µm fiter to remove potentially presented un-dissolved components using nitrogen overpressure. Finally the fill was evacuated for approximately 15 minutes to remove dissolved gas from the solution.

### Gelatin preparation:

Water, glycerol, propylene glycol and sorbitol in appropriate bin were heated up to 88°C. Gelatine and glycine were transferred in to the bin and temperature was maintained at approx. 77.5°C for 20 minutes and slowly mixed. Then the gelatine was de-aerated while being vigorously mixed to achieve final viscosity in the range 19,000 - 23,000 mPa.s. In the end the gelatine solution was maintained melted in the bin heated up to 60°C until it processing to soft gelatine capsules.

### Encapsulation:

Using standard encapsulation machine (for instance: Sankyo, Bochang, Pharmagel, Technophar) from the melted gelatin two gelatin ribbons were prepared by pouring melted gelatin on to lubricated cooling drums. Drums can be cooled by different media - besides air also various liquids are suitable to make a convection of excess heat away. Ribbons with precise thickness were then transferred into cutting dierolles and a welding wedge equipped with a filling nozzle, where capsules were cut off the ribbons, filled with 150 ± 10 mg of the fill and welded. Fresh capsules were then pre-dried and polished with paraffin oil in tumbler dryer for approx 3 hours. Pre-dried capsules were then transferred on plates and dried for 3 days in a drying room (RH < 20%; temperature < 25°C) to achieve content of water in the fill <10%. Dried capsules were optically controlled and then washed in appropriate solvent (n-heptan, ethanol, etc.) to remove polishing agent from the surface.

3-oval capsules (oval shape) weighing 257.7 mg, each containing 1.56 mg of rasagiline mesylate were manufactured. They were subsequently coated with a single layer of the following Eudragit-based coat.

### Eudragit coat - composition:

| Component | Weight Percent (%) |
|---|---|
| Glyceryl monostearate | 1.12 |
| Tween 80 | 0.45 |
| Polyethylene glycol 20,000 | 3.00 |
| NaOH | 0.20 |
| Eudragit L30D55 | 49.7 |
| Purified water | 45.6 |

### Coat preparation:

Appropriate portion of water was pre-heated up to 80°C, then glyceryl monostearate was added together with Tween 80 and mixed for approx 10 minutes (approx. 4000 rpm) until smooth emulsion was achieved. Meanwhile, in a separate vessel, 1 part of Polyethylene glycol 20,000 was mixed with approx 5 parts of water and homogenized for 20 minutes (approx. 150 rpm). Eudragit L30 D55 was poured into appropriate container and slowly neutralized with 1N NaOH. After that, the solution containing glyceryl monostearate was added, homogenized for a while and then PEG 20,000 solution was added to complete the coating solution. When it was completed, the admixture was homogenized for approx 20 minutes (approx 250 rpm).

### Coating:

Coating was performed in RAMA COTA coater equipped with standard Glatt spray gun. Inlet air 40±5°C, outlet air 30±5°C, atomization air pressure 3±1 bar, drum speed 12 ±3 rpm. The capsules were sprayed at a low spray rate, of 2-3g/ min.kg. The coating was applied in various thicknesses.

The dissolution in 0.1 N HCl was determined, 500 ml, 37°C, 75 rpm; apparatus 2 with sinkers.

| Coating code | % weight gain of capsules | % release in 2 hr HCl |
|---|---|---|
| C3 | 2.40 | 63.3 |
| C2 | 3.6 | 25.9 |
| C1 | 5.90 | 28.9 |
| C5* | 6.0 | 27.8 |
| C4 | 9.20 | 20.9 |

| | | |
|---|---|---|
| * C5 was dried prior coating to achieve water content in fill < 8%. | | |

These capsules were not resistant to acid conditions, even when the coating was increased to 9.20% by weight, and even when dried under additional drying conditions.

Microscopic examination showed that the lack of acid resistance stems from pinholes in the apical area of the oval capsules, which is the part of the capsule having the most mechanical strain.

### Example 7: Rasagiline Soft Gelatin Capsules Comprising Additional Solubilizing Agents

Gelatin Capsules were prepared using the following fill:

| Component | % |
|---|---|
| Rasagiline mesylate | 1.560 |
| Cremophor RH 40 | 32.321 |
| Polyglycerol-3- oleate | 32.821 |
| BHA | 0.500 |
| PEG 400 | ad 100.000 |

The gelatin coating was made from the following excipients Gelatin composition:

| Component | % |
|---|---|
| Gelatin | 45.20 |
| Glycerol 85 | 20.00 |
| Sorbitol 70% sol | 4.00 |
| Glycine | 0.45 |
| Purified Water | 30.35 |

Gelatin capsules were prepared in the same manner as described in Example 6.

3-round capsules weighing (248.9 mg) were manufactured. The capsules were round to prevent mechanical stress and creation of the pinholes during dissolution which can cause early leakage. Round shape of capsules also helps to get more uniform coat on the capsules.

Enteric coating: These capsules were subsequently coated with either a single layer 8% by weight, based on Eudragit coat (the same as described in Example 6), or by a double layer, which is 4% of Hypromellose 2910 (Pharmacoat 606) and 4% Eudragit.

### Hypromellose coat - composition:

| Component | % |
|---|---|
| Hypromellose 2910 | 6.00 |
| Purified water | ad 100.00 |

The dissolution percent in 0.1 N HCl was determined. The capsules were in 0.1 N HCl for 120 minutes, then subsequently transferred to phosphate buffer at a pH of 6.8.

Dissolution was performed in USP Dissolution apparatus 1 (Basket). First 2 hours in 500 ml 0.1 M HCl, then in Dissolution Medium II: 500 ml Buffer pH 6.8. The buffer pH 6.8 is prepared as follows: dissolve 27.22 g of KH2PO4 in water and dilute with water to 1000ml; place 250ml of this solution in a 1000mL volumetric flask add 112mL of aqueous NaOH (0.2M), then add water to volume. Time for Dissolution medium II: 90 minutes; stirring rate: 75 rpm.

| Dissolution % | 0 | 120 | 125 | 135 | 150 | 165 | 180 | 220 | 240 |
|---|---|---|---|---|---|---|---|---|---|
| double layer | 0 | 3,0 | 3,2 | 3,3 | 5,1 | 16,2 | 34,5 | 86,5 | 93,6 |
| single layer | 0 | 19,1 | 19,4 | 19,7 | 20,5 | 21,1 | 22,3 | 61,6 | |

The results show that a single layer coat, even while added at 8% weight, was not sufficient to prevent dissolution of Rasagiline Mesylate in acidic conditions. In both the single layer and double layer coating, dissolution in the pH of 6.8 medium was not rapid. After 60 minutes in phosphate buffer at a pH of 6.8, the dissolution was less than 25% for both single layer and double layer coating.

This indicates that in order to achieve fast dissolution in aqueous neural pH, it is beneficial to use a fill based on primarily hydrophilic excipients.

In addition, these capsules showed some dissolution in 0.1N HCl after 120 minutes. The amount of dissolution in the single layer was greater than in the double layer, indicating that a primary non-enteric coating (subcoat) applied directly to the gelatin coating is beneficial in enhancing the acid resistance of the capsules.

### Example 8: Rasagiline Soft Gelatin Capsules

Gelatin Capsules were prepared using the following fill:

| Component | Weight | % |
|---|---|---|
| Rasagiline Mesylate | 130 g | 1.04 |
| Glycerin, 85% | 735.29 g | 5.88 |
| PEG 400 | 11633.46g | 93.07 |
| BHA | 1.25g | 0.01 |

### Gelatin Composition

| Component | Weight | % |
|---|---|---|
| Gealtina 150 bloom, var. B, | 67.8 kg | 45.20 |
| Glycerol 85% PhEur | 30 kg | 20.00 |
| Sorbitol 70%, noncrystalline PhEur | 6 kg | 4.00 |
| Purified Water | 38.5197 kg | 30.35 |

Capsules were prepared in the same way and under the same conditions as described in Example 6.

The dissolution percent in 0.1 N HCl was determined. The capsules were in 0.1 N HCl for 120 minutes, then subsequently transferred to phosphate buffer at a pH of 6.8.

Dissolution was performed in USP Dissolution apparatus 1 (Basket). First 2 hours in 500 ml 0.1 M HCl, then in Dissolution Medium II: 500 ml Buffer pH 6.8. The buffer pH 6.8 is prepared as follows: dissolve 27.22 g of KH2PO4 in water and dilute with water to 1000ml; place 250ml of this solution in a 1000mL volumetric flask add 112mL of aqueous NaOH (0.2M), then add water to volume. Time for Dissolution medium II: 90 minutes; stirring rate: 75 rpm.

| Dissolution Percent | 0 | 120 | 125 | 135 | 150 | 165 | 180 | 220 | 240 |
|---|---|---|---|---|---|---|---|---|---|
| Double layer | 0 | 5,4 | 5,4 | 5,4 | 13,5 | 21,0 | 60,9 | 93,7 | 94,5 |
| Single layer | 0 | 0,0 | 0,1 | 2,0 | 6,0 | 12,9 | 15,2 | 60,5 | |

### Example 9: Rasagiline Soft Gelatin Capsules

### Components:

| Component | Function | Quantity |
|---|---|---|
| *Fill* | | |
| Rasagiline Mesylate | Active substance | 1.560 mg |
| Polyethylene glycol 400 | Solvent | 139.580 mg |
| Glycerol 85% | Co-solvent; hydration | 8.831 mg |
| Buthylhydroxyanisol | Antioxidant | 0.030 mg |

| *Capsule* | | |
|---|---|---|
| Gelatin 150 bloom, type B | Gel forming agent | 45.557 mg |
| Glycerol 85% | Plasticizer | 20.158 mg |
| Sorbitol 70% (non cristallising) | Plasticizer | 4.032 mg |
| Glycine | Cross-linking inhibitor, and Glycine/NaOH buffer | 2.50 mg |
| Sodium hydroxide | Glycine/NaOH buffer | 0.04 mg |
| Purified Water | Processing Agent | 28.260 mg |

| *Coatings* | | |
|---|---|---|
| Hypromelose | Coating 1 | 5.488 mg |
| Eudragit L30 D-55 | Coating 2 | 8.810 mg |
| Sodium hydroxide | pH adjustment | 0.120 mg |
| Glycerol monostearate | Glidant | 0.659 mg |
| Tween 80 | Emulsifier | 0.263 mg |
| Polyethylene Glycol 20,000 | Plasticizer | 1.761 mg |

The gelatin shell was made from the following excipients Gelatin composition:

| Component | % |
|---|---|
| Gelatin | 45.20 |
| Glycerol 85 | 20.00 |
| Sorbitol 70% sol | 4.00 |
| Sodium Hydroxide | 0.04 |
| Glycine | 2.5 |
| Purified Water | 28.26 |

The capsules are prepared in 5 steps: fill preparation, gelatin preparation, encapsulation and drying, coating and packaging.

### Fill preparation:

Under ambient condition in appropriate container Polyethylene glycol 400, Glycerol 85% and Buthylhydroxyanizol were mixed. Rasagiline Mesylate was added and the mixture was mixed for approx. 30 min to attain a clear yellowish solution. Then the solution was filtrated through a 5 µm filter to remove un-dissolved components using nitrogen overpressure. The solution was then evacuated for approx. 15 minutes to remove dissolved gas from the solution

### Gelatin preparation:

Part of water, glycerol and sorbitol were heated up to 88°C in an appropriate bin. Glycine was dissolved in remaining portion of water and pH of the solution was adjusted with sodium hydroxide to pH 8.0+/-0.1. Gelatin was then transferred in to the bin and temperature is maintained at approx. 77.5°C for 20 minutes and the mixture was slowly mixed. The solution was de-aerated whilst vigorously mixing to achieve final viscosity in the range 19,000 - 23,000 mPa.s. The solution was maintained melted in the bin heated up to 60°C.

### Encapsulation:

Using a standard encapsulation machine (Sankyo, Jp), 2 gelatin ribbons were prepared by pouring melted gelatin onto lubricated cooling drums. Ribbons with precise thickness were then transferred into cutting die-rolls and welding wedge equipped with a filling nozzle, where capsules were cut off the ribbons, filled with 150 ± 10 mg of the fill and welded shut.

Fresh capsules were pre-dried and polished with paraffin oil in tumbler dryer for approx 3 hours (with temperature not exceeded 30°C). Pre-dried capsules were transferred on plates and dried for 3 days in a drying room (RH < 20%; temperature < 25°C) to achieve content of water in the fill <10%.

Dried capsules were visually checked and then washed in n-heptan to remove polishing agent from the surface.

### Coating:

Coating was performed in Glatt GMPC II machine. Capsules were transferred in to a coating drum pre-heated up to 37°C. Hypromelose dissolved in water was used as the 1st coat (desired spray rate > 4 g/min.kg or >20 g/min for 5kg batch). The desired thickness of the 1st coat is approx 2-3% by weight of the capsules.

After the first coat the capsules were dried for max 60 minutes and 37°C.

As a second coat, Eudragit L30D 55 dispersion with plasticizer and glidant was applied; (desired spray rate > 4 g/min.kg or >20 g/min for 5kg batch); and a desired thickness of the second coat of approx 2.8 - 3.8 % by weight, preferably 3.3% by weight was applied (total thickness of 2 coats all together is approx. 5.8 ± 20%).

Capsules were dried for max 60 minutes and 37°C and then cooled down below 30°C. Coated capsules were subjected to optical control to remove defective capsules.

### Packaging

Coated capsules were packed into desired packaging, preferably into packaging which ensures moisture protection. Al/Al blisters are preferred, however also other types of moisture protection packs are suitable - following examples of packaging possibilities illustrate (without limitation) additional possibilities: container/closure systems consisting of containers made from various types of material (glass, HDPE, PP, PE, PS, PVC, PVdC, Al etc) with appropriate closure system optionally also equipped with moisture controlling device and optionally additional moisture controlling device enclosed into the container, blister systems consisting from two foils usually made from HDPE, PP, PE, PS, PVC, PVdC, All materials optionally packed in additional moisture protecting container or foil pack.

### Stability:

Al/Al blisters were packed with capsules after 1 month in accelerated conditions of 40°C at 75% relative humidity. The capsules were found to have 0.10% total impurities, and a Rasagiline Assay of between 90.0-110.0%.

### Dissolution:

The dissolution percent of the capsules was determined. The capsules were in 0.1 N HCl for 120 minutes, then subsequently transferred to phosphate buffer at a pH of 6.2.

Dissolution was performed in USP Dissolution apparatus 1 (Basket). First 2 hours in 500 ml 0.1 M HCl, then in Dissolution Medium II: 500 ml Buffer pH 6.2. The buffer pH 6.2 is prepared as follows: dissolve 20,415g of KH₂PO₄ in water and 121,5 ml 0.2M NaOH and in volumetric flask add water to volume 3000ml. Time for Dissolution medium II: 90 minutes; stirring rate: 75 rpm.

| Time (minutes) | 0 | 120 | 130 | 140 | 150 | 165 | 180 | 210 |
|---|---|---|---|---|---|---|---|---|
| Dissolution (%) | 0 | 0 | 2.82 | 14.33 | 26.44 | 94.07 | 95.68 | 95.72 |

The dissolution percent of the capsules was determined. The capsules were in 0.1 N HCl for 120 minutes, then subsequently transferred to phosphate buffer at a pH of 6.8.

Dissolution was performed in USP Dissolution apparatus 1 (Basket). First 2 hours in 500 ml 0.1 M HCl, then in Dissolution Medium II: 500 ml Buffer pH 6.8. The buffer pH 6.8 is prepared as follows: dissolve 27.22 g of KH2PO4 in water and dilute with water to 1000ml; place 250ml of this solution in a 1000mL volumetric flask add 112mL of aqueous NaOH (0.2M), then add water to volume. Time for Dissolution medium II: 90 minutes; stirring rate: 75 rpm.

| Time (minutes) | 0 | 120 | 130 | 140 | 150 | 165 | 180 | 210 |
|---|---|---|---|---|---|---|---|---|
| Dissolution (%) | 0 | 0 | 5.00 | 22.23 | 85.04 | 93.76 | 94.06 | 94.02 |

This formulation was found to resist dissolution in 0.1 N HCl for 120 minutes, and to dissolve quickly in pH 6.2 and 6.8 conditions almost entirely within 45 minutes.

### Example 10: Dissolution of enteric-coated tablets and capsules with improved dissolution characteristics

The following tables compare the results of dissolution with and without modification of the gelatin shell.

**Table 11a: Typical time-course of dissolution of enteric-coated tablets with improved dissolution characteristics - by the use of a proper plasticizer and subcoat**

| **Buffer phosphate pH 6.2** | | | | | | |
|---|---|---|---|---|---|---|
| **Dissolution results - TABLETS** | | | | | | |
| **75 rpm, 37°C, 500 ml, Basket** | | | | | | |
| **2 hr in 0.1 N HCI** | **0 for all 4 tablets** | | | | | |
| | **Time (min.)** | | | | | |
| | **15** | **30** | **45** | **60** | **75** | **90** |
| **Tablet 1** | **1** | **2** | **67** | **103** | **106** | **107** |
| **Tablet 2** | **3** | **3** | **38** | **89** | **100** | **101** |
| **Tablet 3** | **2** | **2** | **35** | **91** | **100** | **100** |
| **Tablet 4** | **1** | **9** | **60** | **99** | **100** | **101** |
| mean | 2.00 | 3.00 | 45.00 | **91.00** | 100 | 101.00 |

The cores of tablets tested in Table 11a were prepared as described in the Example 1 with the ingredients in the following table:

### Tablet Cores:

| **Ingredients** | **Quantity per tablet (mg)** |
|---|---|
| Rasagiline | 1.56 |
| Manitol | 79.89 |
| Aerosil 200 | 0.6 |
| Starch NF | 10.0 |
| Starch STA-RX-1500 | 10.0 |
| Stearic Acid | 2.0 |

The cores of the tablets were then coated with an enteric coating according to following table:

### Enteric Coating:

| **Ingredients** | **Quantity per tablet (mg)** |
|---|---|
| Talc | 2.0 |
| Pharmacoate606 G | 4.8 |
| Eudragit L-100-55 | 6.25 |
| Talc extra fine | 3.1 |
| Triethyl citrate | 1.25 |

**Table 11b: Typical time-course of dissolution of enteric-coated capsules with improved dissolution characteristics - by the use of a proper plasticizer and subcoat - and WITHOUT modification of the gelatin shell (no increase of ionic strength, no buffering)**

| **Buffer phosphate pH 6.2 Dissolution results - CAPSULES, double-coated, NO BLUFFER in gel** | | | | | | |
|---|---|---|---|---|---|---|
| **75 rpm, 37°C, 500 ml, Basket** | | | | | | |
| **2 hr in 0.1 N HCI** | **0 for all 6 capsules** | | | | | |
| | **Time (min.)** | | | | | |
| | **20** | **30** | **45** | **60** | **75** | **90** |
| Capsule 1 | 12.91 | 19.97 | **90.54** | 95.36 | | 95.541 |
| Capsule 2 | 17.75 | 65.53 | **94.54** | 95.49 | | 95.584 |
| Capsule 3 | 12.89 | 23.81 | **92.46** | 95.55 | | 95.665 |
| Capsule 4 | 12.31 | 43.51 | **94.62** | 95.90 | | 96.001 |
| Capsule 5 | 0.00 | 14.63 | **23.30** | 49.72 | | 93.91 |
| Capsule 6 | 12.33 | 20.04 | **88.45** | 94.23 | | 95.344 |
| mean | 12.61 | 21.93 | **91.50** | 95.43 | | 95.56 |

The capsules tested in Table 11b were prepared as described in the Example 7.

**Table 11c: Typical time-course of dissolution of enteric-coated capsules with improved dissolution characteristics - by the use of a proper plasticizer and subcoat - and WITH modification of the gelatin shell (WITH increase of ionic strength, buffering to pH 8.5)**

| **Buffer phosphate pH 6.2 Dissolution results - CAPSULES, double coated, BUFFERRED GEL** | | | | | | |
|---|---|---|---|---|---|---|
| **75 rpm, 37°C, 500 ml, Basket** | | | | | | |
| **2 hr in 0.1 N HCI** | 0 for all 6 capsules | | | | | |
| | Time (min.) | | | | | |
| | **20** | **30** | **45** | **60** | **75** | **90** |
| Capsule 1 | 25.44 | **87.85** | 94.23 | 94.41 | | 94.49 |
| Capsule 2 | 14.11 | **90.45** | 93.69 | 93.91 | | 93.84 |
| Capsule 3 | 14.34 | **89.45** | 95.48 | 95.59 | | 95.61 |
| Capsule 4 | 15.61 | **92.29** | 94.69 | 94.96 | | 94.95 |
| Capsule 5 | 19.23 | **89.41** | 93.26 | 93.58 | | 93.74 |
| Capsule 6 | 23.32 | **89.68** | 92.45 | 92.52 | | 92.64 |
| mean | 17.42 | **89.56** | 93.96 | 94.16 | | 94.16 |

The capsules tested in Table 11c were prepared as described in the Example 9.

The results in the above tables show that the use of cross-linking inhibitor in the gelatin shell in combination with inorganic base, together forming a buffer of alkalic pH (e.g. pH 8.5) and increasing ionic strength of the gel solution, helps in achieving faster disintegration and dissolution of enteric-coated soft gelatin capsules.

### Discussion

In general, when delayed release formulations are compared to their immediate release counterparts in bio-studies, the Cₘₐₓ of the delayed release formulations are lower than the Cₘₐₓ in the corresponding immediate release formulations. As illustrated in the above Examples 1-10, achieving a delayed-release pharmaceutical formulation in which the Cₘₐₓ is similar to the corresponding immediate-release formulation is not trivial.

It has been surprisingly found from the results of Examples 1-9 that the formulations of the current invention meet the criteria of bioequivalence to the known, immediate release rasagiline mesylate formulations in a single dose bio-equivalence study in healthy subjects. These criteria include similarity of Cₘₐₓ and AUC₀₋ₜ (area under the curve) within the range of 80-125% within a 90% confidence interval between the new formulations and the known, immediate release formulations.

As shown in the results of Example 10, it has also been surprisingly found that the use of cross-linking inhibitor helps to achieve faster disintegration and dissolution of enteric-coated soft gelatin capsules. However, the role of cross-linking inhibitor used herein is not only prevention of cross-linking reaction. In combination with weak organic acids and inorganic salts, cross-linking inhibitor modifies the pH and ionic strength of the liquid phase in the gelatin gel. Specifically, it has been found that increased ionic strength and higher buffer capacity of the gelatin shell causes the migration of solvent from the dissolution media to the gelatin shell and/or inner coating layer, which is eroding, swelling and disintegrating faster than without such a modification.

Even more surprisingly, it has been found that that increased ionic strength and higher buffer capacity of the (inner) gelatin shell causes not only the migration of solvent from the dissolution media to the gelatin shell and/or inner coating layer, but also the formation of swelled gel-liquid phase at higher pH level, which assists the dissolution of the outer coat. As a result, not only the disintegration of the gelatin shell is facilitated, but the onset of dissolution of the whole coating system is much more rapid.

## Claims

1. A pharmaceutical composition comprising a liquid fill which includes an amount of rasagiline mesylate, a shell comprising gelatin surrounding the liquid fill, and an enteric coating surrounding the shell.

2. The pharmaceutical composition of claim 1, wherein the liquid fill further comprises a hydrophilic or amphiphilic solvent or surfactant.

3. The pharmaceutical composition of claim 2, wherein the hydrophilic or amphiphilic solvent or surfactant is selected from the group consisting of: polyethylene glycol, propylene glycol, polyoxyethylene sorbitan fatty acid esters, polyoxyethylene castor oil derivatives and ethanol.

4. The pharmaceutical composition of claim 3, wherein the hydrophilic solvent is polyethylene glycol, preferably polyethylene glycol 400.

5. The pharmaceutical composition of any one of claims 1-4 which is free of propylene glycol.

6. The pharmaceutical composition of any one of claims 1-5, wherein the liquid fill further comprises an anti-oxidant, preferably a water-soluble anti-oxidant.

7. The pharmaceutical composition of claim 6 wherein the antioxidant is selected from the group consisting of: propyl gallate, BHA, BHT and ascorbic acid, preferably BHA.

8. The pharmaceutical composition of any one of claims 1-7 wherein the shell further comprises a plasticizer, preferably selected from the group consisting of glycerol, sorbitol and a combination thereof.

9. The pharmaceutical composition of any one of claims 1-8 wherein the enteric coating comprises Poly(methacrylic acid, ethyl acrylate) 1 : 1.

10. The pharmaceutical composition of claim 9 wherein the enteric coating further comprises a plasticizer, preferably polyethylene glycol 20,000.

11. The pharmaceutical composition of any one of claims 1-10 further comprising a non-enteric subcoat, preferably comprising hydroxypropyl methyl cellulose, which is present between the gelatin shell layer and the enteric coating layer, wherein preferably the total weight of the subcoat and enteric coating layer is less than 10% of the total capsule weight.

12. The pharmaceutical composition of claim 11 wherein the weight of the enteric coating layer is less than 8% of the total capsule weight, preferably less than 6%, more preferably less than 4%, of the total capsule weight.

13. The pharmaceutical composition of claim 11 or 12 wherein the gelatin shell layer comprises a cross-linking inhibitor, preferably glycine.

14. The pharmaceutical composition of claim 13 wherein when placed in a basket apparatus in 500 mL of aqueous buffered solution at a pH of 8.2 at 75 revolutions per minute, not less than 85% of the rasagiline is released into solution within 30 minutes.

15. The pharmaceutical composition of claim 1 wherein when placed in a basket apparatus in 500 mL of aqueous 0.1 N HCl at 37°C at 75 revolutions per minute, not more than 10% of the rasagiline is released into solution in 120 minutes and when the composition is subsequently placed in a basket apparatus in 500 mL of aqueous buffered solution at a pH of 6.8 at 37°C at 75 revolutions per minute, not less than 75% of the rasagiline is released into solution within 90 minutes, or
when the composition is subsequently placed in a basket apparatus in 500 mL of aqueous buffered solution at a pH of 5.2 at 37°C at 75 revolutions per minute, not more than 10% of the rasagiline is released into solution within 90 minutes, or
when the composition is subsequently placed in a basket apparatus in 500 mL of aqueous buffered solution at a pH of 6.2 at 37°C at 75 revolutions per minute, not less than 75% of the rasagiline is released into solution within 45 minutes.

## Patentansprüche

1. Pharmazeutische Zusammensetzung, umfassend eine flüssige Füllung, die eine Menge von Rasagilinmesylat enthält, eine Hülle umfassend Gelatine die flüssige Füllung umschließend und ein magensaftresistenter die Hülle umschließender Überzug.

2. Pharmazeutische Zusammensetzung nach Anspruch 1, wobei die flüssige Füllung weiterhin ein hydrophiles oder amphiphiles Lösungsmittel oder Tensid umfasst.

3. Pharmazeutische Zusammensetzung nach Anspruch 2, wobei das hydrophile oder amphiphile Lösungsmittel oder Tensid ausgewählt ist aus der Gruppe bestehend aus: Polyethylenglykol, Propylenglykol, Polyoxyethylensorbitan-Fettsäureestern, Polyoxyethylen-Rizinusölderivaten und Ethanol.

4. Pharmazeutische Zusammensetzung nach Anspruch 3, wobei das hydrophile Lösungsmittel Polyethylenglykol, vorzugsweise Polyethylenglykol 400 ist.

5. Pharmazeutische Zusammensetzung nach einem der Ansprüche 1 bis 4, die frei von Polyethylenglykol ist.

6. Pharmazeutische Zusammensetzung nach einem der Ansprüche 1 bis 5, wobei die flüssige Füllung weiterhin ein Antioxidans, vorzugsweise ein wasserlösliches Antioxidans umfasst.

7. Pharmazeutische Zusammensetzung nach Anspruch 6, wobei das Antioxidans ausgewählt ist aus der Gruppe bestehend aus Propylgallat, BHA, BHT und Ascorbinsäure, vorzugsweise BHA.

8. Pharmazeutische Zusammensetzung nach einem der Ansprüche 1 bis 7, wobei die Hülle weiterhin einen Weichmacher umfasst, vorzugsweise ausgewählt aus der Gruppe bestehend aus Glyzerin, Sorbitol und einer Kombination hiervon.

9. Pharmazeutische Zusammensetzung nach einem der Ansprüche 1 bis 8, wobei der magensaftresistente Überzug Poly(methacrylsäure, Ethylacrylat) 1:1 umfasst.

10. Pharmazeutische Zusammensetzung nach Anspruch 9, wobei der magensaftresistente Überzug weiterhin einen Weichmacher, vorzugsweise Polyethylenglykol 20.000 umfasst.

11. Pharmazeutische Zusammensetzung nach einem der Ansprüche 1 1 bis 10, weiterhin umfassend einen nicht-magensaftresistenten Grundüberzug, vorzugsweise umfassend Hydroxypropylmethylcellulose, welcher zwischen der Gelatine-Hüllschicht und der magensaftresistenten Überzugsschicht vorhanden ist, wobei vorzugsweise das Gesamtgewicht des Grundüberzugs und der magensaftresistenten Überzugsschicht weniger als 10% des Gesamtgewichts der Kapsel beträgt.

12. Pharmazeutische Zusammensetzung nach Anspruch 11, wobei das Gewicht der magensaftresistenten Überzugsschicht weniger als 8% des Gesamtgewichts der Kapsel, vorzugsweise weniger als 6%, besonders bevorzugt weniger als 4% des Gesamtgewichts der Kapsel beträgt.

13. Pharmazeutische Zusammensetzung nach Anspruch 11 oder 12, wobei die Gelatine-Hüllschicht einen Vernetzungsinhibitor, vorzugsweise Glycin umfasst.

14. Pharmazeutische Zusammensetzung nach Anspruch 13, wobei wenn in einer Korbvorrichtung in 500 mL wässriger, gepufferter Lösung bei einem pH von 8.2 bei 75 Umdrehungen pro Minute platziert, nicht weniger als 85% des Rasagilins innerhalb von 30 Minuten in die Lösung freigesetzt wird.

15. Pharmazeutische Zusammensetzung nach Anspruch 1, wobei wenn in eine Korbvorrichtung in 500 mL wässriges 0,1 N HCl bei 37°C bei 75 Umdrehungen pro Minute platziert, nicht mehr als 10% des Rasagilins innerhalb von 120 Minuten in die Lösung freigesetzt wird und
wenn die Zusammensetzung nachträglich in eine Korbvorrichtung in 500 mL wässriger, gepufferter Lösung bei einem pH von 6,8 bei 37°C. bei 75 Umdrehungen pro Minute platziert wird, nicht weniger als 75% des Rasagilins innerhalb von 90 Minuten in die Lösung freigesetzt wird, oder
wenn die Zusammensetzung nachträglich in eine Korbvorrichtung in 500 mL wässriger, gepufferter Lösung bei einem pH von 5,2 bei 37°C bei 75 Umdrehungen pro Minute platziert wird, nicht mehr als 10% des Rasagilins innerhalb von 90 Minuten in die Lösung freigesetzt wird, oder
wenn die Zusammensetzung nachträglich in eine Korbvorrichtung in 500 mL wässriger, gepufferter Lösung bei einem pH von 6,2 bei 37°C bei 75 Umdrehungen pro Minute platziert wird, nicht weniger als 75% des Rasagilins innerhalb von 45 Minuten in die Lösung freigesetzt wird.

## Revendications

1. Composition pharmaceutique comprenant une charge liquide qui inclut une quantité de mésylate de rasagiline, une enveloppe comprenant de la gélatine entourant la charge liquide, et un enrobage gastrorésistant et entérosoluble entourant l'enveloppe.

2. Composition pharmaceutique selon la revendication 1, dans laquelle la charge liquide comprend de plus un solvant ou un tensioactif hydrophile ou amphiphile.

3. Composition pharmaceutique selon la revendication 2, dans laquelle le solvant ou tensioactif hydrophile ou amphiphile est choisi dans le groupe constitué de : polyéthylèneglycol, propylèneglycol, esters de polyoxyéthylènesorbitan et d'acide gras, dérivés de polyoxyéthylène-huile de ricin et éthanol

4. Composition pharmaceutique selon la revendication 3, dans laquelle le solvant hydrophile est du polyéthylèneglycol, de préférence du polyéthylèneglycol 400.

5. Composition pharmaceutique selon l'une quelconque des revendications 1 à 4 qui est exempte de propylèneglycol.

6. Composition pharmaceutique selon l'une quelconque des revendications 1 à 5, dans laquelle la charge liquide comprend de plus un antioxydant, de préférence un antioxydant hydrosoluble.

7. Composition pharmaceutique selon la revendication 6, dans laquelle l'antioxydant est choisi dans le groupe constitué de : propylgallate, BHA, BHT et acide ascorbique, de préférence BHA.

8. Composition pharmaceutique selon l'une quelconque des revendications 1 à 7, dans laquelle l'enveloppe comprend de plus un plastifiant de préférence choisi dans le groupe constitué de glycérol, de sorbitol et d'une combinaison de ceux-ci.

9. Composition pharmaceutique selon l'une quelconque des revendications 1 à 8, dans laquelle l'enrobage gastrorésistant et entérosoluble comprend du poly(acide méthacrylique, acrylate d'éthyle) 1:1.

10. Composition pharmaceutique selon la revendication 9, dans laquelle l'enrobage gastrorésistant et entérosoluble comprend de plus un plastifiant, de préférence du polyéthylèneglycol 20 000.

11. Composition pharmaceutique selon l'une quelconque des revendications 1 à 10, comprenant de plus une sous-couche qui n'est pas entérique, de préférence comprenant de l'hydroxypropylméthylcellulose, qui est présente entre la couche de l'enveloppe de gélatine et la couche d'enrobage gastrorésistant et entérosoluble, où de préférence le poids total de la sous-couche et de la couche d'enrobage gastrorésistant et entérosoluble est de moins de 10 % du poids total de la capsule.

12. Composition pharmaceutique selon la revendication 11, dans laquelle le poids de la couche d'enrobage gastrorésistant et entérosoluble est de moins de 8 % du poids total de la capsule, de préférence de moins de 6 %, de manière plus particulièrement préférée de moins de 4 %, du poids total de la capsule.

13. Composition pharmaceutique selon la revendication 11 ou 12, dans laquelle la couche de l'enveloppe de gélatine comprend un inhibiteur de réticulation, de préférence de la glycine.

14. Composition pharmaceutique selon la revendication 13, dans laquelle, quand elle est mise dans un dispositif à panier dans 500 ml de solution aqueuse tamponnée à un pH de 8,2 à raison de 75 tours par minute, pas moins de 85 % de la rasagiline est libérée dans la solution en moins de 30 minutes.

15. Composition pharmaceutique selon la revendication 1, dans laquelle, quand elle est mise dans un dispositif à panier dans 500 ml de HCl 0,1 N aqueux à 37°C à raison de 75 tours par minute, pas plus de 10 % de la rasagiline est libérée dans la solution en 120 minutes et quand la composition est ultérieurement mise dans un dispositif à panier dans 500 ml de solution aqueuse tamponnée à un pH de 6,8 à 37°C à raison de 75 tours par minute, pas moins de 75 % de la rasagiline est libérée dans la solution en moins de 90 minutes, ou
quand la composition est ultérieurement mise dans un dispositif à panier dans 500 ml de solution aqueuse tamponnée à un pH de 5,2 à 37°C à raison de 75 tours par minute, pas plus de 10 % de la rasagiline est libérée dans la solution en moins de 90 minutes, ou
quand la composition est ultérieurement mise dans un dispositif à panier dans 500 ml de solution aqueuse tamponnée à un pH de 6,2 à 37°C à raison de 75 tours par minute, pas moins de 75 % de la rasagiline est libérée dans la solution en moins de 45 minutes.
